(19)

Europäisches
Patentamt
European
Patent Office
Office européen
des brevets

(11)  **EP 2 666 760 A1**

(12)  **EUROPEAN PATENT APPLICATION**
published in accordance with Art. 153(4) EPC

(43) Date of publication:
**27.11.2013 Bulletin 2013/48**

(21) Application number: **12734199.8**

(22) Date of filing: **11.01.2012**

(51) Int Cl.:
**C07C 2/34** $^{(2006.01)}$   **C07C 11/02** $^{(2006.01)}$
**C07B 61/00** $^{(2006.01)}$

(86) International application number:
**PCT/JP2012/000098**

(87) International publication number:
**WO 2012/096158 (19.07.2012 Gazette 2012/29)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.01.2011  JP 2011004912**

(71) Applicant: **Idemitsu Kosan Co., Ltd.**
**Chiyoda-ku**
**Tokyo 100-8321 (JP)**

(72) Inventors:
• **INAGAKI, Junichi**
**Ichihara-shi**
**Chiba 299-0193 (JP)**

• **MACHIDA, Masashi**
**Ichihara-shi**
**Chiba 299-0193 (JP)**
• **OKANO, Masaki**
**Ichihara-shi**
**Chiba 299-0193 (JP)**
• **TSUJI, Minako**
**Ichihara-shi**
**Chiba 299-0193 (JP)**

(74) Representative: **Vossius & Partner**
**Siebertstrasse 4**
**81675 München (DE)**

(54)  **PROCESS FOR PRODUCTION OF ALPHA-OLEFIN UNSATURATED DIMER**

(57)    A method for producing an unsaturated dimer by reacting an α-olefin having 6 to 14 carbon atoms in an agitated vessel, wherein a metallocene-based catalyst is used as a reaction catalyst; and the supply flow rate of hydrogen ($V_H$) to the vessel is 0.2 to 1.2 [(NL/hr) per L of olefin] and the agitation power ($P_V$) is 0.18 KW/m$^3$ or more of the time of reaction.

**EP 2 666 760 A1**

**Description**

Technical Field

The invention relates to a method for producing an $\alpha$-olefin unsaturated dimer.

Background Art

A poly $\alpha$-olefin (PAO) obtained by polymerizing 1-octene, 1-decene and 1-dodecene is used in the field of a lubricant oil base of an engine or a transmitter, a viscosity index improver, an antiwear additive, a friction modifier, ink, paint, emulsion and a mold releasing agent for toner, or the like.
PAO has several viscosity levels which are required according to application. As for the method for controlling viscosity, a method in which a polymer with a specific polymerization degree can be selectively obtained by selecting a polymerization catalyst or a method in which fractions differing in polymerization degree are separated after polymerization.
On the other hand, a dimer of $\alpha$-olefin can be produced in the same manner as in the case of PAO, and is used for raw materials of a plasticizer of a resin or the like. Since it is used as a raw material, it is preferred that a dimer of $\alpha$-olefin be a dimer having a reactive group (a vinylidene group). Therefore, as the standard for judging the quality thereof, the remaining ratio of an unsaturated bond (vinylidene) is used.
As the method for producing a PAO, Patent Documents 1 to 3 each discloses a method in which an $\alpha$-olefin is polymerized by using a metallocene catalyst, for example. In the method disclosed in these documents, hydrogen is supplied during the reaction in order to enhance the activity of the metallocene catalyst. In addition to the effect of enhancing the catalytic activity, hydrogen also has an effect of converting the unsaturated bond of the resulting polymer to a single bond. The above-mentioned publications mainly refer to a method for producing a PAO to be used for lubrication oil. Reducing the unsaturated bond of the polymer is preferable in respect of improving thermal stability of lubricant oil.
On the other hand, in the production of an unsaturated dimer of an $\alpha$-olefin, use of hydrogen is not necessarily preferable since hydrogen lowers the remaining ratio of an unsaturated bond. However, there is a problem that, without using hydrogen, the activity of the metallocene is deteriorated to lower the reaction activity.

Related Art Documents

Patent Documents

    Patent Document 1: JP-A-2001-335607
    Patent Document 2: WO2010/074233
    Patent Document 3: WO2010/053022

SUMMARY OF THE INVENTION

The invention is aimed at providing a method for producing an $\alpha$-olefin unsaturated dimer in a high yield at a sufficiently high reaction rate on the industrial scale.
As a result of intensive studies, the inventors have found that, in the polymerization reaction of an $\alpha$-olefin, by controlling the hydrogen supply flow rate ($V_H$) and the agitation power ($Pv$) to a specific level, a desired dimer can be produced in a high yield at a high reaction rate. The invention has been made on this finding.
According to the invention, the following method for producing an unsaturated dimer of an $\alpha$-olefin is provided.

    1. A method for producing an unsaturated dimer by reacting an $\alpha$-olefin having 6 to 14 carbon atoms in an agitated vessel, wherein a metallocene-based catalyst is used as a reaction catalyst; and the supply flow rate of hydrogen ($V_H$) to the vessel is 0.2 to 1.2 [(NL/hr) per L of olefin] and the agitation power ($Pv$) is 0.18 KW/m$^3$ or more of the time of reaction.
    2. The method for producing an unsaturated dimer according to 1, wherein the supply flow rate of hydrogen ($V_H$) is 0.2 to 0.8 [(NL/hr) per L of olefin] and the agitation power ($Pv$) is set to 0.7 KW/m$^3$ or more.
    3. The method for producing an unsaturated dimer according to 1 or 2, wherein the $\alpha$-olefin is selected from 1-octene, 1-decene and 1-dodecene.
    4. The method for producing an unsaturated dimer according to 1 or 2, wherein the
    $\alpha$-olefin is 1-decene.

According to the production method of the invention, an unsaturated dimer of an α-olefin can be produced efficiently in a high yield.

MODE FOR CARRYING OUT THE INVENTION

In the method for producing an unsaturated dimer of an α-olefin of the invention, in an agitated vessel, α-olefins having 6 to 14 carbon atoms are allowed to react with each other while stirring, thereby to produce a dimer.

Examples of an α-olefin having 6 to 14 carbon atoms inlude 1-hexene, 1-octene, 1-decene, 1-dodecene and 1-tetradecene. Preferable α-olefins include 1-octene, 1-decene and 1-dodecene, with 1-decene being particularly preferable.

The α-olefin may be used in combination of two or more or may be used singly.

In the invention, a metallocene-based catalyst is used as the reaction catalyst. As the metallocene-based catalyst used in the invention, a catalyst disclosed in WO2010/053022 (Patent Document 3) can be preferably used. Specifically, a metallocene-based catalyst containing a transition metal compound represented by the following formula (1) and an aluminum-containing co-catalyst (B) is used in the invention.

$$\{C_5R^1R^2R^3R^4(A^1R^aR^bR^C)\}\{C_5R^5R^6R^7R^8(A^2R^dR^eR^f)\}MXY \qquad (1)$$

wherein $R^1$ to $R^8$, $(A^1R^aR^bR^c)$ and $(A^2R^dR^eR^f)$ are independently a substituent which is bonded to a cyclopentadienyl group. $R^1$ to $R^8$ are independently a hydrogen atom or a hydrocarbon group having 1 to 10 carbon atoms; and $R^a$ to $R^f$ are independently a hydrocarbon group having 1 to 10 carbon atoms. Two or more groups selected from $R^a$, $R^b$ and $R^c$ may be bonded with each other to form a ring; two or more groups selected from $R^d$, $R^e$ and $R^f$ may be bonded with each other to form a ring; $A^1$ and $A^2$ are independently an element belonging to the 14th group of the periodic table of the elements; M is a transitional metal belonging to the 4th group of the periodic table of elements; and X and Y are independently a covalent ligand or an ionic ligand.

Further, the metallocene-based catalyst is a metallocene-based catalyst containing a transitional metal compound represented by the following formula (2) and methylaluminoxane.

$$(RC_5H_4)_2MX_2 \qquad (2)$$

wherein R is a hydrogen atom or a hydrocarbon group having 1 to 10 carbon atoms, M is a transitional metal belonging to the 4th group of the periodic table of elements; and X is a covalent ligand or an ionic ligand.

Of the transitional metal compounds represented by the above formulas (1) and (2), bis-t-butylcylopentadienylzirconium dichloride is particularly preferable.

As the co-catalyst (B) containing aluminum, methylaluminoxane is preferable.

By allowing an α-olefin to be reacted by using the above-mentioned metallocene-based catalyst, a mixture containing a component larger than a trimer in addition to a dimer is obtained. Therefore, in the invention, a reaction product is fractionated by distillation or the like, whereby dimer components are recovered.

In the invention, no particular restrictions are imposed on the polymerization method. Any of bulk polymerization, solution polymerization, suspension polymerization, slurry polymerization, vapor phase polymerization or the like can be used. As for polymerization conditions, the polymerization temperature is normally 0 to 200°C, preferably 30 to 150°C, more preferably 40 to 120°C. As for the amount ratio of the raw material olefin to the catalyst, it is preferred that the raw material olefin/the transition metal compound (molar ratio) be 1 to $10^8$, particularly 100 to $10^5$. The polymerization time is normally 5 minutes to 20 hours, the reaction pressure is preferably from normal pressure to 0.2 MPaG, with from normal pressure to 0.1 MPaG being particularly preferable.

In the production method of the invention, it is preferred that production be conducted without using a solvent. However, a solvent may be used. In this case, aromatic hydrocarbons such as benzene, toluene, xylene and ethylbenzene; alicyclic hydrocarbons such as cyclopentane, cyclohexane and methylcyclohexane; aliphatic hydrocarbons such as pentane, hexane, heptane and octane and halogenated hydrocarbons such as chloroform and dichloromethane can be used. These solvents may be used alone or in combination of two or more.

In the invention, a preliminary polymerization can be conducted by using a polymerization catalyst. A preliminary polymerization can be conducted by allowing a small amount of olefin to contact the catalyst components. No specific restrictions are imposed on this method, and a known method can be used. No specific restrictions are imposed on an olefin used in a preliminary polymerization. For example, ethylene, an α-olefin having 3 to 20 carbon atoms or a mixture thereof can be given. It is advantageous to use the same olefin as that of a raw olefin used in this polymerization. A preliminary polymerization temperature is normally -20 to 200°C, preferably -10 to 130°C, and more preferably 0 to 80°C. In the preliminary polymerization, as a solvent, inactive hydrocarbons, aliphatic hydrocarbons, aromatic hydrocarbons, monomers or the like can be used. Of these, aliphatic hydrocarbons and aromatic hydrocarbons are preferable.

Further, a preliminary polymerization may be conducted without using a solvent. In the preliminary polymerization, it is preferable to adjust the conditions such that the amount of a preliminary polymerization product per mill mole of transition metal components in a catalyst becomes 1 to 10,000g, in particular, 1 to 1,000g.

In the production method of the invention, when a raw olefin is reacted, catalytic activity is improved by supplying hydrogen in the reaction system, whereby the reaction rate (k) is increased. The supply flow rate($V_H$) of hydrogen to a vessel during the reaction is 0.2 to 1.2 [(NL/hr per L of olefin)], preferably 0.3 to 1.0 [(NL/hr per L of olefin)], and particularly preferably 0.7 to 0.8 [(NL/hr per L of olefin)]. If $V_H$ is smaller than 0.2 [(NL/hr) per L of olefin], the catalyst activity is not improved, resulting in a lowering of the reaction rate. On the other hand, if $V_H$ is larger than 1.2 [(NL/hr per L of olefin)], the amount of hydrogen in the vessel becomes excessive, and the double bond of a dimer is hydrogenated irrespective of the agitation power.

The unit means the amount of hydrogen supplied (normal liter) per hour relative to per liter (L) of an olefin as a raw material.

In the invention, the agitation power (Pv) of an agitator is 0.18 $KW/m^3$ or more, preferably 0.3 $KW/m^3$ or more, particularly preferably 0.4 $KW/m^3$ or more.

If Pv is smaller than 0.18 $KW/m^3$, catalytic activity may not be improved since hydrogen cannot contact the metallocene catalyst sufficiently.

The upper limit of Pv is not particularly restricted. However, taking the scale of equipment and the size of apparatus into consideration, the upper limit of Pv is normally around 2 $KW/m^3$ or less. It is preferred that Pv be 1.5 $KW/m^3$ or less, with 1.2 $KW/m^3$ being particularly preferable.

The agitation power indicates the power of agitation, and is defined by the agitation power per unit volume (the volume of raw olefin in the reaction chamber).

No specific restrictions are imposed on the vessel or the agitator used in the invention. A reaction vessel or an agitated vessel which is known in this technical field can be used. The supply flow rate of hydrogen (gas flow rate) is preferably 2.5 m/s or more. The supply flow rate of hydrogen can be controlled by adjusting the supply amount or by adjusting the aperture of a hydrogen supply mouth (nozzle or the like).

As the agitation blade, a Pfaudler impeller, a max blend impeller, a turbine blade or the like are preferable.

EXAMPLES

Example 1

A 25,000L-reaction vessel provided with a turbine blade as the agitator was used. To the reaction vessel, 15,000L of 1-decene as raw olefin was supplied. Stirring was conducted for 30 minutes with bubbling with $N_2$.

Next, the pressure inside the vessel was increased to 12 kPaG with $N_2$. The pressure was further increased by 2 kPaG with $H_2$.

Heat medium oil was circulated in a heating jacket, whereby the temperature of 1-decene in the reaction chamber was increased to 40°C and kept constant.

To the reaction vessel, methylaluminoxane (MAO) as the co-catalyst and bis-t-butylcyclopentadienylzirconium dichloride $(t\text{-}BuCp)_2ZrCl_2$ (hereinafter referred to as T2) was added. MAO was added in an amount of 4000 $\mu$mol/(per L of 1-decene) and T2 was added in an amount of 40 $\mu$mol/(per L of 1-decene). The reaction temperature was 40°C, the agitation power was 0.30 $kW/m^3$ and the hydrogen flow was 0.20 [(NL/hr/per L of 1-decene].

Thereafter, a sample was recovered every hour to calculate the reaction rate. The reaction time was 7.0 hours.

Comparative Example 1

A dimer of 1-decene was produced in the same manner as in Example 1, except that the agitation power and the hydrogen flow were changed to the values shown in Table 1.

The reaction rate constant k and the vinylidene purity under the conditions in Example 1 and Comparative Example 1 were measured. The results are shown below.

Table 1

| | Agitation power Pv | Hydrogen flow $V_H$ | Reaction rate constant k ($min^{-1}$) | Vinylidene purity (%) |
|---|---|---|---|---|
| Example1 | 0.30 | 0.20 | 0.0068 | 91.4 |

(continued)

|  | Agitation power Pv | Hydrogen flow $V_H$ | Reaction rate constant k (min$^{-1}$) | Vinylidene purity (%) |
|---|---|---|---|---|
| Com. Ex. 1 | 0.30 | 0.01 | 0.0013 | 95.3 |
| Pv: [kW/m$^3$]<br>$V_H$: [(NL/hr) per L of 1-decene] | | | | |

· Reaction rate constant k

The reaction rate constant k was obtained by the following formula:

$$k = -\ln(C/C_0)/t$$

(wherein k is a reaction rate constant, C is a raw material concentration after the reaction, $C_0$ is a raw material concentration before the reaction, and t is a reaction time)

· Vinylidene purity

Measured by gas chromatography (GC main body: GC2010, manufactured by Shimadzu Corporation). The conditions are as follows.

(1) Column: Ultra 2,25 m $\times$ 0.2 mm $\times$ 0.33 $\mu$m, MAX. 325°C
(2) Flow

·Import pressure: 207.5 kPa
·Column flow: 1.37 ml/min
·Linear velocity: 41.7 cm/s
·Split ratio: 30.0
·Total flow: 47.4 ml/min
·Injection mode: SPLIT
·Control mode: Linear velocity
·Carrier gas: He
·FID/INJ=300°C/300°C
·Column: Temperature programmed meter 42 mim
·Injection amount: 1 $\mu$l
·Temperature elevation pattern: Hold at 100°C for 1 minute, and elevated at a rate of 10°C per minute for 20 minutes to be 300°C, followed by measurement at 300 °C

Industrial Applicability

The invention is suitable as the method for producing an unsaturated dimer of an $\alpha$-olefin.

Although only some exemplary embodiments and/or examples of this invention have been described in detail above, those skilled in the art will readily appreciate that many modifications are possible in the exemplary embodiments and/or examples without materially departing from the novel teachings and advantages of this invention. Accordingly, all such modifications are intended to be included within the scope of this invention.

The documents described in the specification are incorporated herein by reference in its entirety.

**Claims**

1. A method for producing an unsaturated dimer by reacting an $\alpha$-olefin having 6 to 14 carbon atoms in an agitated vessel, wherein a metallocene-based catalyst is used as a reaction catalyst; and the supply flow rate of hydrogen ($V_H$) to the vessel is 0.2 to 1.2 [(NL/hr) per L of olefin] and the agitation power (Pv) is 0.18 KW/m$^3$ or more of the time of reaction.

2. The method for producing an unsaturated dimer according to claim 1, wherein the supply flow rate of hydrogen ($V_H$)

is 0.2 to 0.8 [(NL/hr) per L of olefin] and the agitation power (Pv) is set to 0.7 KW/m$^3$ or more.

3. The method for producing an unsaturated dimer according to claim 1 or 2, wherein the α-olefin is selected from 1-octene, 1-decene and 1-dodecene.

4. The method for producing an unsaturated dimer according to claim 1 or 2, wherein the α-olefin is 1-decene.

<table>
<tr><td colspan="3" align="center">**INTERNATIONAL SEARCH REPORT**</td><td colspan="2">International application No.<br>PCT/JP2012/000098</td></tr>
</table>

A.   CLASSIFICATION OF SUBJECT MATTER
*C07C2/34*(2006.01)i, *C07C11/02*(2006.01)i, *C07B61/00*(2006.01)n

According to International Patent Classification (IPC) or to both national classification and IPC

B.   FIELDS SEARCHED

Minimum documentation searched (classification system followed by classification symbols)
C07C2/34, C07C11/02, C07B61/00

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2012 |
| Kokai Jitsuyo Shinan Koho | 1971–2012 | Toroku Jitsuyo Shinan Koho | 1994–2012 |

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
|---|---|---|
| X | WO 2006/088020 A1  (Idemitsu Kosan Co., Ltd.),<br>24 August 2006 (24.08.2006),<br>claims; paragraphs [0025] to [0027]; examples 3, 5<br>& JP 2006-225348 A      & US 2009/0069614 A1<br>& EP 1849757 A1 | 1-4 |
| A | JP 2010-534762 A  (Exxonmobil Chemical Patents Inc.),<br>11 November 2010 (11.11.2010),<br>claims; examples<br>& US 2009/0036725 A1      & EP 2173779 A2<br>& WO 2009/017953 A2      & CN 101809042 A | 1-4 |

[X]   Further documents are listed in the continuation of Box C.          [ ]   See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>   14 March, 2012 (14.03.12) | Date of mailing of the international search report<br>   27 March, 2012 (27.03.12) |
|---|---|
| Name and mailing address of the ISA/<br>   Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2012/000098 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2002-532249 A  (Phillips Petroleum Co.),<br>02 October 2002 (02.10.2002),<br>claims; examples<br>& US 2001/0053742 A1      & EP 1152829 A1<br>& WO 2000/037175 A1 | 1-4 |

Form PCT/ISA/210 (continuation of second sheet) (July 2009)

**EP 2 666 760 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001335607 A
- WO 2010074233 A
- WO 2010053022 A